# FASCICULE DE BREVET EUROPEEN

(11) **EP 0 783 307 B1**
(45) Date de publication et mention de la délivrance du brevet: **20.02.2002**
(21) Numéro de dépôt: 95931260.4
(22) Date de dépôt: 19.09.1995
(51) Int. Cl.: A61K 31/505, A61P 17/02

(54) **UTILISATION DU 2,4-DIAMINO PYRIMIDINE 3-OXYDE OU DE L'UN DE SES SELS DANS LE TRAITEMENT DES DESORDRES DE LA MATURATION ET DE LA STRUCTURATION DU COLLAGENE**
VERWENDUNG VON 2,4-DIAMINOPYRIMIDIN-3-OXYD UND/ODER SEINE SALZE ZUR BEHANDLUNGVON STÖRUNGEN DER KOLLAGENREIFUNG ODER -STRUKTURIERUNG
USE OF 2,4-DIAMINO PYRIMIDINE 3-OXIDE OR A SALT THEREOF FOR TREATING COLLAGEN MATURATION AND STRUCTURING DISORDERS

(30) Priorité: 19.09.1994 FR 9411133
(43) Date de publication de la demande: 16.07.1997
(73) Titulaire: L'OREAL, 75008 Paris (FR)
(72) Inventeur: MAHE, Yann, F-91390 Morsang-sur-Orge (FR); BRETON, Lionel, F-78000 Versailles (FR); GALEY, Jean-Baptiste, F-93600 Aulnay-sous-Bois (FR); BERNARD, Bruno, F-92200 Neuilley-sur-Seine (FR)
(74) Mandataire: Stalla-Bourdillon, Bernard
(86) Numéro de dépôt international: FR9501197
(87) Numéro de publication internationale: WO9609048

(56) Documents cités:
- EP-A- 0 548 883
- WO-A-92/01437
- INVEST. OPHTHALMOL. VISUAL SCI., vol. 34, no. 3, 1993 pages 567-575, J.T. HANDA ET AL. 'Minoxidil inhibits ocular cell proliferation and lysyl hydroxylase activity.'
- INVEST. OPHTHALMOL. VISUAL SCI., vol. 35, no. 2, 1994 pages 463-469, J.T. HANDA ET AL. 'Inhibition of cultured human RPE cell proliferation and lysyl hydroxylase activity by hydrogen derivatives of minoxidil.'

## Description

La présente invention a pour objet l'utilisation, en tant que substance active, du 2,4-diamino pyrimidine 3-oxyde ou de l'un de ses sels pour la préparation d'une composition thérapeutique, à usage pharmaceutique ou vétérinaire, destinée au traitement des désordres de la maturation et de la structuration du collagène.

L'obtention de faisceaux de procollagène natif requiert l'hydroxylation d'un certain nombre de résidus prolyl et lysyl du préprocollagène au sein du réticulum endoplasmique rugueux, puis la glycosylation des résidus hydroxy-lysyls dans l'appareil de Golgi. Cette maturation du collagène a fait l'objet de nombreuses études.

Ainsi, le Minoxidil, à savoir le 2,4-diamino-6-pipéridino pyrimidine 3-oxyde, déjà décrit et utilisé dans le traitement de certaines hypertensions et dans le traitement de l'alopécie androgénique, a fait l'objet de nombreuses études en tant qu'inhibiteur spécifique de la lysyl hydroxylase, enzyme intervenant dans la maturation du collagène.

A ce sujet, les études de Murad et al. publiées dans Arch. of Biochem. Biophys., Vol.292, n°1, p.234-238 (1992) et Vol.308, n°1, p.42-47 (1994), ont en effet permis de montrer que la capacité à inhiber la lysyl hydroxylase nécessitait la présence d'un groupe amine tertiaire situé en para de la fonction N-oxyde du noyau 2,4-diamino pyrimidine et que de préférence, ce groupe devait être un groupe pipéridine comme dans le Minoxidil.

Or, on a maintenant constaté de façon surprenante et tout à fait inattendue que le 2,4-diamino pyrimidine 3-oxyde ou 2,4-DPO (dans la suite), bien que ne comportant pas de groupe amine tertiaire en para de la fonction N-oxyde du noyau 2,4-diamino pyrimidine, permettait d'inhiber de façon spécifique la lysyl hydroxylase.

Ce composé a déjà été décrit dans WO-92/01437 en tant qu'agent actif destiné au traitement cosmétique, par application topique, de la chute des cheveux. Cependant, contrairement au Minoxidil, le 2,4-DPO ne possède aucun effet anti-hypertenseur et présente donc une innocuité particulièrement satisfaisante, tout en ayant une activité inhibitrice de la lysyl hydroxylase égale voire même supérieure à celle du Minoxidil.

La présente invention a donc pour objet l'utilisation, en tant que substance active du 2,4-DPO ou de l'un de ses sels pour la préparation d'une composition thérapeutique, à usage pharmaceutique ou vétérinaire, destinée au traitement des désordres de la maturation du collagène chez l'homme ou chez l'animal.

Dans la suite, l'expression "2,4-DPO" signifiera non seulement le 2,4-DPO lui-même mais également l'un de ses sels tels que définis ci-après.

Par désordre de la maturation du collagène, on doit entendre selon l'invention, toute accumulation non désirée de collagène, et notamment, tout désordre de la structure de la matrice collagénique du tissu cutané ou de la conjonctive oculaire ; ces désordres peuvent être d'origine spontanée, traumatique ou post-opératoire. Parmi ces désordres, on peut notamment citer les vitréorétinopathies, les sclérodermites systémiques, les épaississements cutanés induits par les ultra-violets et les chéloïdes cicatriciels.

L'utilisation du 2,4-DPO selon l'invention permet donc d'obtenir notamment une amélioration esthétique du patient traité.

Si, selon l'invention, le traitement peut être curatif, il est néanmoins de préférence utilisé de façon préventive, c'est-à-dire au cours de la période durant laquelle se produit la structuration et, par conséquent, l'accumulation de collagène, ceci ayant lieu notamment pendant la période de cicatrisation.

Le 2,4-DPO peut être utilisé pour la préparation d'une composition thérapeutique se présentant sous une forme administrable par voie topique, transdermique ou intradermique.

Par sels physiologiquement acceptables, on doit entendre selon l'invention, des sels d'addition d'un acide tels que ceux de l'acide sulfurique, chlorhydrique, phosphorique, acétique, benzoïque, salicylique, glycolique, acéturique, succinique, nicotinique, tartrique, maléïque, méthane sulfonique, picrique et lactique.

Lorsque la composition thérapeutique se présente sous une forme administrable par voie topique, la proportion en 2,4-DPO est généralement comprise entre 0,0001 et 5 % en poids, et de préférence comprise entre 0,01 et 2 % en poids par rapport au poids total de la composition.

Le véhicule peut être de nature très variée mais est de préférence un milieu physiologiquement acceptable pour une application topique, compatible bien entendu avec la substance active.

Le 2,4-DPO peut se trouver dans ce milieu soit à l'état dissous, soit à l'état dispersé, notamment sous forme micronisée.

Le milieu physiologiquement acceptable peut être constitué par de l'eau ou un mélange d'eau et d'un solvant ou un mélange de solvants. Parmi les solvants pouvant être utilisés, on peut citer notamment les alcools inférieurs en C₁-C₄ comme l'éthanol, l'isopropanol, le tertiobutanol, les alkylène glycols, les alkyléthers d'alkylène glycol et de dialkylène glycol tels que le monoéthyléther d'éthylène glycol, le monométhyléther de propylène glycol, le monoéthyléther de diéthylène glycol. Ces compositions destinées à une application topique peuvent également contenir au moins un additif conventionnel ou un principe actif.

Parmi ceux-ci, on peut notamment citer les filtres UV.A et UV.B comme les méthoxycinnamates et les dérivés de benzophénone, les anti-oxydants et/ou capteurs de radicaux libres tels que le DMSO, l'α-tocophérol, le BHA, le BHT, la superoxyde dismutase, les agents hydratants tels que l'urée, la glycérine, l'acide lactique, les hydroxyacides, la thiamorpholinone et ses dérivés et les dérivés de l'acide pyrrolidone carboxylique notamment ses sels de sodium et de potassium, les agents anti-inflammatoires stéroïdiens et non-stéroïdiens tels que l'hydrocortisone, la β-méthasone, la dexaméthasone, l'acide acétyl salicylique, l'indométhacine, l'isoprofène, la vitamine D ou l'acide niflumique, et les anti-bactériens tels que ceux appartenant au groupe des macrolides, des pyranosides et des tétracyclines telles que l'érythromycine.

Ces compositions peuvent également contenir des agents conservateurs, des agents stabilisants, des agents régulateurs de pH, des agents modificateurs de pression osmotique et des agents émulsifiants.

Ces compositions destinées à une application topique à base de 2,4-DPO peuvent se présenter sous différentes formes telles que par exemple sous forme de lotions, de gels, de mousses, de dispersions vésiculaires, de sprays ou de mousses aérosol.

Ces compositions peuvent également se présenter sous forme de compositions gélifiées ou épaissies. Parmi les compositions gélifiées, on peut en particulier citer celles, essentiellement aqueuses, gélifiées par des hétérobiopolysaccharides tels que la gomme de xanthane, les scléroglucanes ou les dérivés de cellulose et en particulier les éthers de cellulose, ou les compositions hydroalcooliques gélifiées par des polyhydroxyéthylacrylates ou méthacrylates. Parmi les compositions aqueuses épaissies, on peut citer celles obtenues en particulier à l'aide d'un acide polyacrylique réticulé par un agent polyfonctionnel tel que ceux commercialisés sous les dénominations de "Carbopol®" par la Société Goodrich. Il est cependant bien entendu possible d'utiliser comme agent épaississant tout agent habituellement employé en pharmacie ou en dermo-pharmacie.

Par voie topique, le 2,4-DPO est généralement appliqué sur les parties du corps à traiter à raison de 0,0001 à 5 mg/kg/jour et de préférence de 0,001 à 1 mg/kg/jour. Le traitement est généralement poursuivi pendant toute la période pendant laquelle se produit l'accumulation de collagène et peut être éventuellement poursuivi pendant 90 jours après celle-ci.

On peut également, selon l'invention, utiliser le 2,4-DPO pour la préparation de compositions thérapeutiques destinées à une administration par voie transdermique.

La proportion en 2,4-DPO est alors généralement comprise entre 0,001 et 5 % et de préférence comprise entre 0,01 et 2 % par rapport au poids total de la composition.

L'excipient utilisé dans ces compositions est généralement choisi parmi les copolymères ou copolymères vinyliques, additionnés éventuellement d'un promoteur de pénétration.

Les compositions destinées à une application transdermique peuvent être notamment administrées à l'aide d'un système auto-collant adhérant sur la peau et assurant le passage continu, pendant au moins 24 heures, d'une quantité contrôlée de substance active, à travers la peau, dans la circulation sanguine.

Parmi ces systèmes, on peut notamment citer ceux décrits dans la demande FR-88.11685 (2.635.979) ainsi que ceux commercialisés par la Société Tilderm.

Le 2,4-DPO peut être également utilisé pour la préparation de compositions thérapeutiques destinées à une administration par voie intradermique.

La proportion en 2,4-DPO est alors généralement comprise entre 0,0001 et 5 % en poids, et de préférence comprise entre 0,01 et 1 % en poids par rapport au poids total de la composition.

Le 2,4-DPO est alors sous forme soluble dans un véhicule physiologiquement acceptable tel que du sérum physiologique.

Les compositions destinées à une administration intradermique peuvent comprendre en outre différents agents thérapeutiques tels que par exemple des agents antibactériens, des agents antioxydants et des anti-inflamatoires stéroïdiens et non stéroïdiens.

Le 2,4-DPO est généralement administré par voie intradermique à raison d'une dose inférieure ou égale à 5 mg/kg/jour, et de préférence comprise entre 0,0001 et 1 mg/kg/jour. Le traitement est généralement appliqué pendant toute la période durant laquelle se produit l'accumulation de collagène et peut être éventuellement poursuivi pendant 30 jours après celle-ci.

Bien qu'il ait été plus particulièrement fait référence ci-dessus à des compositions destinées à un usage pharmaceutique, il va de soi que celles-ci peuvent être formulées sous une forme plus appropriée pour un usage vétérinaire notamment dans le traitement d'équidés tels que le cheval.

Les compositions selon l'invention quel que soit le mode d'administration ou l'usage auquel elles sont destinées sont préparées selon des méthodes connues.

On va maintenant donner à titre d'illustration les résultats d'études mettant en évidence l'efficacité du 2,4-DPO ainsi que divers exemples de compositions thérapeutiques.

### I - Etude de l'inhibition de l'expression des ARNm codant pour la lysyl hydroxylase (LH)

A partir d'une culture de fibroblastes provenant de gaines conjonctives externes de cheveu humain au cinquième repiquage, on ensemence 2.10⁵ fibroblastes dans des boîtes de Pétri de 60 mm de diamètre contenant un milieu de culture de type "Eagle", modifié Dulbecco et supplémenté par 2 mM de L-glutamine, 1 mM de pyruvate de sodium, 100 unités/ml de pénicilline G, 100 µg/ml de streptomycine S, 250 mg/ml d'amphotérine et 10 % de sérum de veau foetal commercialisé par la Société Gibco BRL.

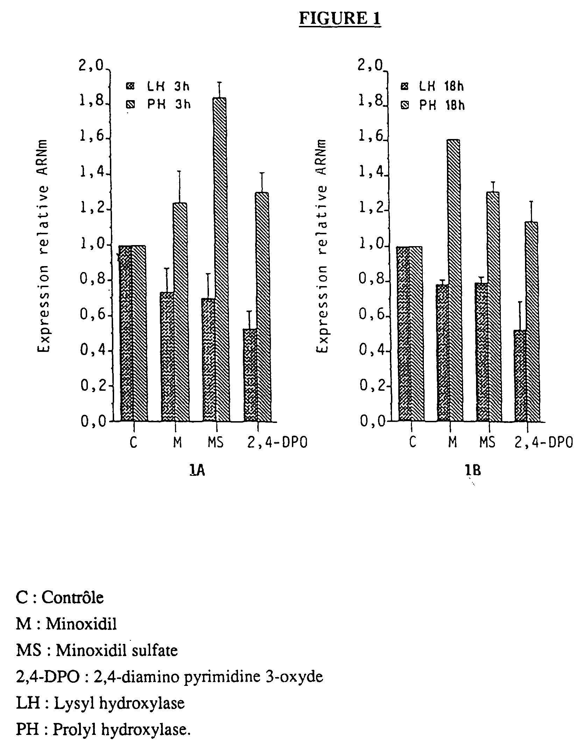

Après 24 heures de culture à 37°C dans une étuve à 95 % air/5 % CO₂, on remplace le milieu par un milieu analogue ne contenant toutefois pas de sérum de veau foetal. On incube ensuite les cellules à 37°C pendant 3 heures (figure 1A) ou 18 heures (figure 1B) en présence de 5 mM de Minoxidil, de 5 mM de 2,4-DPO ou de 5 mM de sulfate de Minoxidil.

Selon la méthode décrite dans "Short protocoles in molecular biology, Harvard medical school J. Willey and sons publisher, 1992", on extrait l'ARN total de chacune des préparations cellulaires puis quantifie celui-ci par mesures spectrophotométriques UV à 280 nm.

Pour permettre l'analyse de l'expression des ARNm, on effectue une amplification de ceux-ci par une réaction de polymérisation en chaîne (PCR).

A partir de 2 µg d'ARN total obtenus précédemment pour chaque préparation cellulaire, on synthétise *in vitro* de l'ADN complémentaire à l'aide d'un kit de transcription réverse commercialisé sous la dénomination de "First Strand C-DNA Synthesis kit" par la Société Pharmacia LKB Biotechnology AB. Une fraction aliquote de l'ADN complémentaire obtenu précédemment est ensuite amplifiée par la méthode de PCR proprement dite en présence des amorces spécifiques de la lysyl hydroxylase (LH) et de la prolyl hydroxylase (PH) suivantes : amorce LH sens : 5'GAAATGGGCCATGTGAGAGCG3' amorce LH antisens : 5'TGTGGATGACAATAGTCGGCACG3' amorce PH sens : 5'GTGGATGGAACAAGCCCTAAGGC3' amorce PH antisens : 5'CCTCCCCACGGCACAGCATTTCG3', pour un volume final de 50 µl.

20 µl du mélange obtenu après PCR sont déposés sur un gel d'électrophorèse à 2 % d'agarose en présence de 0,5 µg/ml de bromure d'éthidium et du tampon d'électrophorèse Tris acétate EDTA (TAE) 1x.

Après migration pendant 1 heure, sous un champ électrique de 100 V, l'intensité des bandes correspondant aux ARNm codant respectivement pour la lysyl hydroxylase et la prolyl hydroxylase est analysée et quantifiée au moyen d'un analyseur d'images et du logiciel commercialisé sous la dénomination de "Bioprofil®" par la Société Vilbert Lourmat.

Selon le même mode opératoire que décrit précédemment, on mesure également les taux d'expression relative des ARNm codant pour la lysyl hydroxylase et pour la prolyl hydroxylase après 3 heures et 18 heures de culture de fibroblastes en absence de tout traitement.

Les résultats sont présentés dans les figures 1A et 1B.

On constate donc que l'incubation de fibroblastes pendant 3 heures en présence de Minoxidil ou de Minoxidil sulfate réduit le taux d'expression relative des ARNm codant pour la lysyl hydroxylase, celui-ci étant alors de 0,75.

Après 3 heures d'incubation en présence de 2,4-DPO, le taux d'expression relative des ARNm codant pour la lysyl hydroxylase est de 0,55.

Le 2,4-DPO inhibe donc plus l'expression des ARNm codant pour la lysyl hydroxylase que le Minoxidil ou que son analogue sulfaté.

On observe des résultats analogues après 18 heures de culture en présence des différents inhibiteurs testés.

Par ailleurs, après 3 heures de culture en présence de Minoxidil ou de Minoxidil sulfate, le taux d'expression relative des ARNm codant pour la prolyl hydroxylase augmente, atteignant respectivement 1,25 et 1,85.

Après 3 heures d'incubation en présence de 2,4-DPO, le taux d'expression relative des ARNm codant pour la prolyl hydroxylase est également plus élevé, celui-ci étant alors de l'ordre de 1,3.

Après 18 heures de culture en présence de Minoxidil, de Minoxidil sulfate ou de 2,4-DPO, les taux d'expression relative des ARNm codant pour la prolyl hydroxylase sont respectivement de 1,6, 1,3 et 1,15.

On n'observe donc pas d'inhibition du taux d'expression relative des ARNm codant pour la prolyl hydroxylase en réponse à ces trois composés.

Les composés testés présentent donc une activité inhibitrice spécifique de la lysyl hydroxylase.

### II - Etude de l'effet du 2,4-DPO sur le réseau de collagène présent dans la matrice extracellulaire de fibroblastes en culture

A une culture primaire de fibroblastes dermiques humains provenant d'une plastie mammaire, dans un milieu DMEM supplémenté par 10 % de sérum de veau foetal, on ajoute 10 µM du 2,4-DPO.

Après trois jours, on lyse les cellules par un choc hypoosmotique et fixe le dépôt de collagène par une solution à 5 % de glutaraldéhyde.

On observe ensuite la matrice extracellulaire des fibroblastes à l'aide d'un microscope électronique à balayage.

On observe également en microscopie électronique à balayage la matrice extracellulaire d'une culture primaire de fibroblastes non traités.

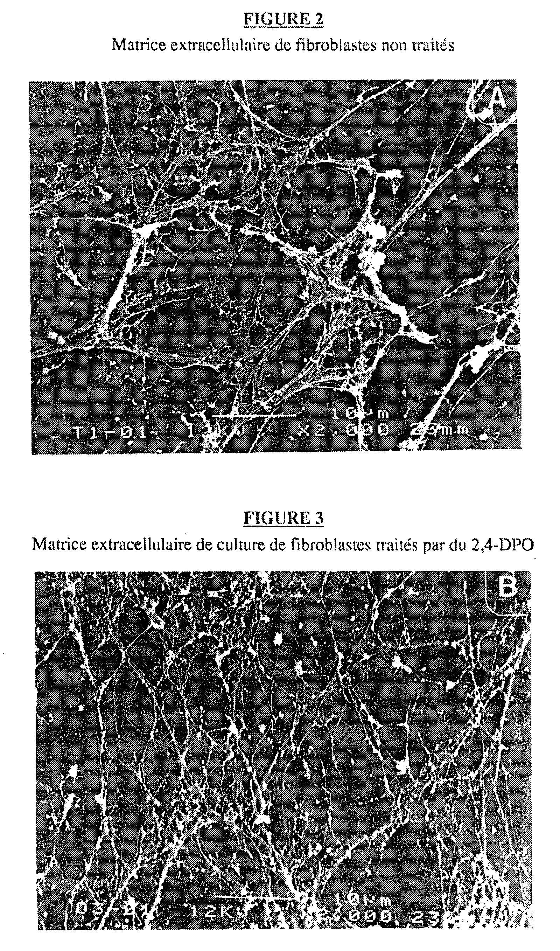

Ces photographies sont présentées dans les Figures 2 et 3.

Comme on peut le constater d'après les photos des Figures 2 et 3, la matrice extracellulaire de fibroblastes non traités présente de gros amas fibreux entourant de larges plages dépourvues de réseau de collagène, tandis que la matrice extracellulaire des fibroblastes traités au 2,4-DPO présente une organisation beaucoup plus régulière dans laquelle on n'observe pas de gros amas fibreux et où la taille des plages dépourvues de réseau de collagène est beaucoup plus réduite.

L'application du 2,4-DPO sur une culture de fibroblastes entraine donc une réorganisation spatiale en micro-réseau des fibres de collagène.

### EXEMPLES DE COMPOSITIONS THERAPEUTIQUES

### EXEMPLE 1 : Crème dermique

On prépare une crème dermique par mélange des ingrédients suivants :
- 2,4-DPO 0,5 g
- Ceteareth 30 7 g
- Stéarate de glycéryle 2 g
- Alcool cétylique 1,5 g
- Polydiméthylsiloxane 1,5 g
- Huile de paraffine 15 g
- Glycérine codex pure 20 g
- Conservateurs q.s.
- Eau déminéralisée q.s.p. 100 g

### EXEMPLE 2 : Lotion dermique à pulvériser

On prépare une lotion dermique à pulvériser par mélange des ingrédients suivants :
- 2,4-DPO 0,25 g
- Ethanol 30 g
- Eau déminéralisée q.s.p. 100 g

### EXEMPLE 3 : Solution injectable

On prépare une solution injectable par voie intradermique par mélange des ingrédients suivants :
- 2,4-DPO 0,7 mg
- Sérum physiologique (NaCl 9 g/H₂O qsp 100 ml) q.s.p. 1 ml

## Revendications

1. Utilisation en tant que substance active du 2,4-diamino pyrimidine 3-oxyde (2,4-DPO) ou de l'un de ses sels physiologiquement acceptables pour la préparation d'une composition thérapeutique, à usage pharmaceutique ou vétérinaire, destinée au traitement des désordres de la maturation et de la structuration du collagène.

2. Utilisation selon la revendication 1, **caractérisée par le fait que** les sels sont choisis parmi ceux d'addition d'un acide pris dans le groupe constitué par l'acide sulfurique, chlorhydrique, phosphorique, acétique, benzoïque, salicylique, glycolique, acéturique, succinique, nicotinique, tartrique, maléïque, méthane sulfonique, picrique et lactique.

3. Utilisation selon l'une quelconque des revendications précédentes, **caractérisée par le fait que** ladite composition se présente sous une forme administrable par voie topique, transdermique ou intradermique.

4. Utilisation selon l'une quelconque des revendications précédentes, **caractérisée par le fait que** ladite composition est sous une forme administrable par voie topique, la proportion en 2,4-DPO étant comprise entre 0,0001 et 5 % en poids par rapport au poids total de la composition.

5. Utilisation selon la revendication 4, **caractérisée par le fait que** la proportion en 2,4-DPO est comprise entre 0,01 et 2 % en poids par rapport au poids total de la composition.

6. Utilisation selon l'une quelconque des revendications 1 à 3, **caractérisée par le fait que** ladite composition est sous une forme administrable par voie transdermique, la proportion en 2,4-DPO étant comprise entre 0,001 et 5 % en poids par rapport au poids total de la composition.

7. Utilisation selon la revendication 6, **caractérisée par le fait que** la proportion en 2,4-DPO est comprise entre 0,01 et 2 % en poids par rapport au poids total de la composition.

8. Utilisation selon l'une quelconque des revendications 1 à 3, **caractérisée par le fait que** ladite composition est sous une forme administrable par voie intradermique, la proportion en 2,4-DPO étant comprise entre 0,0001 et 5 % en poids par rapport au poids total de la composition.

9. Utilisation selon la revendication 8, **caractérisée par le fait que** la proportion en 2,4-DPO est comprise entre 0,01 et 1 % en poids par rapport au poids total de la composition.

10. Utilisation selon l'une quelconque des revendications précédentes, **caractérisée par le fait que** ladite composition est destinée à traiter les désordres de la maturation du collagène chez l'homme.

11. Utilisation selon l'une quelconque des revendications 1 à 9, **caractérisée par le fait que** ladite composition est destinée à traiter les désordres de la maturation du collagène chez l'animal.

12. Utilisation selon la revendication 11, **caractérisé par le fait que** l'animal est un équidé.

## Patentansprüche

1. Verwendung von 2,4-Diaminopyridin-3-oxid (2,4-DPO) oder eines seiner physiologisch annehmbaren Salze als aktive Substanz zur Herstellung einer therapeutischen Zubereitung zur pharmazeutischen oder veterinärmedizinischen Verwendung, bestimmt zur Behandlung von Störungen der Reifung und Strukturierung des Kollagens.

2. Verwendung gemäß Anspruch 1, **dadurch gekennzeichnet, dass** die Salze ausgewählt sind unter den Additionen einer Säure aus der Gruppe bestehend aus Schwefelsäure, Chlorwasserstoffsäure (Salzsäure), Phosphorsäure, Essigsäure, Benzoesäure, Salizylsäure, Glykolsäure, Acetursäure, Bernsteinsäure, Nikotinsäure, Weinsäure, Maleinsäure, Methansulfonsäure, Pikrinsäure und Milchsäure.

3. Verwendung gemäß einem der vorstehenden Ansprüche, **dadurch gekennzeichnet, dass** die Zubereitung in auf topischem Wege, transdermalem Wege oder intradermalem Wege verabreichbarer Form vorliegt.

4. Verwendung gemäß einem der vorstehenden Ansprüche, **dadurch gekennzeichnet, dass** die Zubereitung in auf topischem Wege verabreichbarer Form vorliegt, wobei die Menge an 2,4-DPO zwischen 0,0001 und 5 Gew.-% liegt, bezogen auf das Gesamtgewicht der Zubereitung.

5. Verwendung gemäß Anspruch 4, **dadurch gekennzeichnet, dass** die Menge an 2,4-DPO zwischen 0,01 und 2 Gew.-% liegt, bezogen auf das Gesamtgewicht der Zubereitung.

6. Verwendung nach einem der Ansprüche 1 bis 3, **dadurch gekennzeichnet, dass** die Zubereitung in auf transdermalem Wege verabreichbarer Form vorliegt, wobei die Menge an 2,4-DPO zwischen 0,001 und 5 Gew.-% liegt, bezogen auf das Gesamtgewicht der Zubereitung.

7. Verwendung gemäß Anspruch 6, **dadurch gekennzeichnet, dass** die Menge an 2,4-DPO zwischen 0,01 und 2 Gew.-% liegt, bezogen auf das Gesamtgewicht der Zubereitung.

8. Verwendung gemäß einem der Ansprüche 1 bis 3, **dadurch gekennzeichnet, dass** die Zubereitung in auf intradermalem Wege verabreichbarer Form vorliegt, wobei die Menge an 2,4-DPO zwischen 0,0001 und 5 Gew.-% liegt, bezogen auf das Gesamtgewicht der Zubereitung.

9. Verwendung gemäß Anspruch 8, **dadurch gekennzeichnet, dass** die Menge an 2,4-DPO zwischen 0,01 und 1 Gew.-% liegt, bezogen auf das Gesamtgewicht der Zubereitung.

10. Verwendung gemäß einem der vorstehenden Ansprüche, **dadurch gekennzeichnet, dass** die Zubereitung zur Behandlung von Störungen der Reifung des Kollagens beim Menschen bestimmt ist.

11. Verendung gemäß einem der Ansprüche 1 bis 9, **dadurch gekennzeichnet, dass** die Zubereitung zur Behandlung von Störungen der Reifung des Kollagens beim Tier bestimmt ist.

12. Verwendung gemäß Anspruch 11, **dadurch gekennzeichnet, dass** es sich bei dem Tier um ein Tier der Gattung Pferd (Equidae) handelt.

## Claims

1. Use as an active substance of 2,4-diaminopyrimidine 3-oxide (2,4-DPO) or a physiologically acceptable salt thereof for the preparation of a therapeutic composition for pharmaceutical or veterinary application intended for the treatment of collagen maturation and structuring disorders.

2. Use according to Claim 1, **characterized in that** the salts are chosen from the addition salts of an acid taken from the group consisting of sulphuric, hydrochloric, phosphoric, acetic, benzoic, salicylic, glycolic, aceturic, succinic, nicotinic, tartaric, maleic, methanesulphonic, picric and lactic acids.

3. Use according to either of the preceding claims, **characterized in that** the said composition is presented in a form which can be administered topically, transdermally or intradermally.

4. Use according to any one of the preceding claims, **characterized in that** the said composition is in a form which can be administered topically, the proportion of 2,4-DPO being between 0.0001 and 5% by weight relative to the total weight of the composition.

5. Use according to Claim 4, **characterized in that** the proportion of 2,4-DPO is between 0.01 and 2% by weight relative to the total weight of the composition.

6. Use according to any one of Claims 1 to 3, **characterized in that** the said composition is in a form which can be administered transdermally, the proportion of 2,4-DPO being between 0.001 and 5% by weight relative to the total weight of the composition.

7. Use according to Claim 6, **characterized in that** the proportion of 2,4-DPO is between 0.01 and 2% by weight relative to the total weight of the composition.

8. Use according to any one of Claims 1 to 3, **characterized in that** the said composition is in a form which can be administered intradermally, the proportion of 2,4-DPO being between 0.0001 and 5% by weight relative to the total weight of the composition.

9. Use according to Claim 8, **characterized in that** the proportion of 2,4-DPO is between 0.01 and 1% by weight relative to the total weight of the composition.

10. Use according to any one of the preceding claims, **characterized in that** the said composition is intended for treating collagen maturation disorders in man.

11. Use according to any one of Claims 1 to 9, **characterized in that** the said composition is intended for treating collagen maturation disorders in animals.

12. Use according to Claim 11, **characterized in that** the animal is a member of the Equidae family.
